# EUROPEAN PATENT APPLICATION

(11) **EP 4 411 379 A1**
(43) Date of publication of application: **07.08.2024**
(21) Application number: 23154900.7
(22) Date of filing: 03.02.2023
(51) Int. Cl.: G01N 33/58

(54) **SINGLE- OR MULTI-PLEXED NANOPARTICLE-BASED MICROVOLUME BIOMARKER IMMUNODETECTION BY FLUORESCENCE SPECTROSCOPY**

(71) Applicant: State Research Institute Centre for Innovative Medicine, 08406 Vilnius (LT)
(72) Inventor: Bagdonas, Edvardas, LT-08406 Vilnius (LT); Bernotiene, Eiva, LT-08406 Vilnius (LT); Kalvaityte, Ursule, LT-08406 Vilnius (LT); Kirdaite, Gailute, LT-08406 Vilnius (LT); Kausaite Minkstimiene, Asta, LT-08406 Vilnius (LT); Denkovskij, Jaroslav, LT-08406 Vilnius (LT); Uzieliene, Ilona, LT-08406 Vilnius (LT); Ramanaviciene, Almira, LT-08406 Vilnius (LT); Popov, Anton, LT-08406 Vilnius (LT); Jarockyte, Greta, LT-08406 Vilnius (LT); Karabanovas, Vitalijus, LT-08406 Vilnius (LT); Mobasheri, Ali, LT-08406 Vilnius (LT)
(74) Representative: Zaboliene, Reda

(57) **Abstract**

The purpose of the present invention is to enhance the fluorescent tag or fluorescent tag-based immunoassay detection sensitivity through detachment of FT - immune-complexes from the bottom of the 96-well plate and further using a specific immune-complex degrading solution, diluted in PBS and further transfer to a microplate (reiterative glass slide) for more sensitive measurement in microvolume quantities. The proposed method can be applied for both singleplex and multiplex analyte detection.

## Description

### BACKGROUND OF THE INVENTION

The purpose of the invention is the immunodetection of at least one analyte in the immune-complex using fluorescence spectroscopy and quantitative measurement of the fluorescent tags (FT), including quantum dots (QD), fluorescent dyes, fluorescent microspheres in microvolumes of immune-complex degrading solution with PBS.

Standard immunoassays are used to detect one specific analyte. However, one analyte is not enough to make an early and correct diagnosis for complex diseases such as cancer, diabetes, rheumatoid arthritis (RA) and osteoarthritis (OA), which involve a multitude of biomarkers. The simultaneous screening of a variety of biochemical marker targets in body fluids, using various technologies, such as advanced multiplex assays, ensures the extension of detection limits, cheaper costs, and quick analysis. (E.Bernotiene et al. 2020). Those diseases are multifactorial, comprising multiple simultaneous processes during disease progression, therefore detection of a single marker cannot assure early diagnosis, accurate follow-up of disease progression nor evaluation of treatment efficacy. For example, early OA is usually asymptomatic, so the complex evaluation of biochemical markers of extracellular matrix (ECM) turnover, low-grade inflammation, and other biological processes, might lead to more specific evaluation of the disease endotypes, including the anabolic, catabolic and inflammatory aspects of OA (Mobasheri et al. 2019). In this invention, multicolor fluorescent tags (FTs) conjugates are applied to ensure simultaneous detection of analytes A and/or B. Photoluminescence-based assays are more sensitive than absorbance-based assays (Lakowicz, 2006), as the signal-to-noise ratio increases by measuring photoluminescence instead of absorption. Different types of FT are applied in immunoassays, including QDs, fluorescent dyes, fluorescent microspheres and others (Jarockyte G. et al., 2020; Scholtz L. et al., 2022; Liu J. et al., 2019). In order to be used for multiplexed assay, microspheres have to be encoded to identify the interaction between analytes and spheres. Even though the most well-developed and commercialized encoded microspheres are dye-doped, quantum dots (QDs) and upconversion nanoparticles can be used for fluorescence-encoded microsphere production (Liu et al., 2019). QDs are fluorophores, absorbing photons and re-emitting them at different wavelengths. Semiconductor nanocrystals, also known as QDs, are widely used in biological research as fluorescence imaging tools (Chan et al. 2002; Gao and Nie 2003; Jovin 2003; Yu et al. 2006). Because the surface of QDs are easily modifiable, different antibodies and other biomolecules are simple to attach to them (Bilan R. et al, 2015). Therefore, QDs are highly applicable for multiplexed immunoassays (Jarockyte G. et al. 2020). Technologically similar applications could be used with other fluorescent tags (FT), including fluorescent dyes, fluorescent microspheres (Jarockyte G. et al. 2020, Scholtz L. et al. 2022, Liu J. et al, 2019). QDs show several significant advantages over most organic fluorophore dyes. Their tunable core sizes (1-10 nm) generate a wide range of fluorescence emission peaks with nearly Gaussian emission peaks, which do not show the "shoulders" displayed by the typical emission spectra of organic dyes. QDs show very broad absorption patterns, which can be efficiently excited far from their emission spectra to avoid background scattering.

The calorific spectra of fluorescent signals (emission range from 525 nm, 565 nm, 605 nm, 655 nm, etc.) depend on the type of chosen quantum dots. QDs have a high photoluminescence quantum yield, which allows acquisition of high signal intensities from relatively low concentrations (Grabolle et al., 2009). Moreover, QDs show far greater photostability than that of organic dyes at similar wavelengths (Resch-Genger et al. 2008). Therefore, QDs could work as amplifiers when low concentrations of analyte have to be detected. Finally, QDs, due to their optical and chemical properties, are promising candidates for use in multiplexing, owing to their narrow half-peak width down to 30 nm, wide excitation wavelength, high colour purity, and tunable emission. The combination of these fluorescence characteristics makes QDs a very powerful tool for fluorescence multiplexing, single-molecule tracking (SPT), fluorescence resonance energy transfer (FRET), and high-throughput screening.

The detection of the cluster of differentiation 20 (CD20) antigen, which is a biomarker overexpressed by B-lymphocyte cancer cells, using QLISA (quantum dot-linked immunosorbent assay) was demonstrated (Mansur et al., 2015). The application of QLISA was also reported for the detection of the multifunctional cytokine interleukin 6 (IL-6) (Suzuki et al., 2017). QDs, functionalized with carboxyl groups that have active amine groups, were conjugated with partially reduced antibodies via cross linker to form complexes used for detection. Results demonstrated that using amine-functionalized QDs and reading the fluorescence of the QDs from the bottom of the well, the lowest limit of IL-6 detection would be approximately 50 pg/mL, which is undetectable using a standard ELISA (enzyme-linked immunosorbent assay) method (Suzuki et al., 2017).

It is known that in QLISA experiments, QDs alone amplify the detectable signal due to their physical properties as they have rather low detection limits. However, the dilemma on detection sensitivity still remains. Therefore, different signal resolution amplification methods are investigated. Additional conjugation between QDs and other nanoparticles (e.g., gold, silver, etc.) were reported to intensify the QD signal (Jiménez-López and Llorent-Martinez, 2020; Tobias and Jones, 2019). In all these cases, the analytes of interest were detected using a spectrophotometer in cuvettes or read from the bottom of the well (Suzuki et al., Song et al., 2015).

Noteworthy, application of 8 different separation buffers (pH ranging from 7 to 11) to separate QD-antibody complexes from magnetic beads (MB) has been reported, suggesting that the alkaline detachment buffers are most suitable for of QDs quantification in MB-based QLISA, as lower pH solutions cause QD dissolution in acidic solutions (Zhu et al., 2010). Previously published data on application of QLISA for single or multiplex biomarkers fluorescence spectroscopy experiments, detect analytes of interest in standard 50 - 100 µl volumes of 96-well plates or cuvettes or do not specify the volume in the publication.

CN202010587667 describes a single-molecule immunodetection method based on an upconversion fluorescence probe. The method comprises the steps of: after diluting a detected sample, dropwise adding the diluted detected sample onto the detection substrate, combining the detected substance with a bioactive molecule B, washing the detected substance, dropwise adding a diluted immunofluorescence probe to combine the bioactive molecule A with the detected substance combined with the bioactive molecule B, and washing the detected substance; and placing the treated detected substance under a fluorescence microscope, and counting the number of the immunofluorescence probes to obtain the number of the detected objects. The difference is that no QLISA is performed, no detachment step of immune-complexes for microvolume measurement on a glass microslide is performed. CN202110435783 describes the method for detecting small molecules based on indirect competitive fluorescence ELISA of platinum-coated gold nanoparticles and carbon dots, which comprises the following steps: marking IgG (immunoglobulin G) by using the platinum-coated gold nanoparticles, taking the compound as a signal amplifier, catalyzing an added colour developing agent by the compound to generate a catalytic oxidation product, and quenching fluorescence of the carbon dots introduced into a system, and finally, detecting the change of the fluorescence intensity of the carbon dots by using a multifunctional microplate reader so as to realise the quantitative detection of the small molecular substances. The difference is that in the present invention QLISA is performed, the signal amplification occurs by transfer of a detached immuno-conglomerate with QDs to a microquantity and measured on a plate. There is an additional step of further transfer of diluted immune-complex - PBS solution to a microplate or more sensitive measurement in microvolume quantities is performed.

Present invention enables the enhancement of the reaction sensitivity, (namely, reduces the lowest detection limit) up to forty times.

### SUMMARY OF INVENTION

The purpose of the present invention is to enhance the FT based immunoassay (FT-LISA) detection sensitivity through detachment of multicolor fluorescent tags, and quantification in microvolumes of FT-immune-complex degrading solution, diluted with PBS. Fluorescent tags (FT) include QDs, fluorescent dyes and fluorescent microspheres.

The described invention comprises further steps, namely detachment of streptavidin-coated FTs and biotinylated antibody (Ab) complexes, dilution with PBS (phosphate-buffered saline) at a ratio immune-complex solution / PBS ratio 1:1 - 1:5 and their transfer to microvolume reiterative glass slide-based spectrophotometry format.

The present invention provides *in vitro* method for fluorescence-based detection of at least one analyte by spectroscopy in a sample obtained from a subject which comprises: (i) providing a sample with at least one target analyte on the well bottom of plate, resulting in coating of analyte; (ii) contacting analyte-coated wells with biotinylated detection antibody, wherein said biotinylated detection antibody allows to bind the immune-complex with streptavidin-coated FTs; (iii) forming an immune-complex comprising at least one analyte, biotinylated detection antibody and streptavidin-coated FTs; (iv) applying the immune-complex with the degrading solution for the detachment of FTs from the bottom of the well to solution, which comprises biotinylated antibody - streptavidin-coated FT complexes; (v) diluting of the immune-complex with a buffer or diluent; (vi) transferring the diluted immune-complex - PBS solution to a microplate in microvolume quantities for the assessment of at least one analyte using fluorescence spectroscopy. The analyte is incubated with a biotinylated detection antibody and is followed by addition of streptavidin-coated FTs to the 96-well plate.

In preferred embodiment FTs as photoluminescent immune-complex-QDs conjugates on the bottom of FT-LISA wells of 96-well plate can be detached using a specific immune-complex degrading solution, then diluted with PBS and further transferred to a microplate (reiterative glass slide) for more sensitive measurement in microvolume quantities from 0.5 to 5 µl.

The fluorescent measurement is provided in two ways. The first, the fluorescent measurement of at least one analyte is performed from the bottom of the plate, based on (i) - (iii) steps of the method. The immune-complex comprising at least one analyte, biotinylated detection antibody and streptavidin-coated FTs, is formed in a well plate.

The second, the fluorescent measurement of at least one analyte is performed on glass microslide in microvolume size based on the steps (i) - (vi) of the method. The detachment and transfer of immune-complex to microvolume detection on the glass slide improves the signal and sensitivity of the detection.

The transfer of the well bottom-linked immune-complexes to a liquid phase is performed using an immune-complex degrading solution.

In another embodiment, the method further comprises a step of dilution of immune-complexes with PBS and transferring of said complexes on microslide to microvolume size in amount from 0.5 to 5 µ.

The immune-complex degrading solution was diluted with PBS in ratio 1:1 - 1:5 v/v. wherein the dilution of the immune-complex with PBS improves the surface tension and ensures the formation of round-shaped microdrop of the immune-complex, wherein the drop must be stretched between two glass surfaces.

The diluted FT - immune-complex - PBS solution, which comprises biotinylated antibody - streptavidin-coated FT complexes is evaluated on the reading formats for the FT fluorescence quantitative measurement in microvolumetric size of 0.5 - 5 µl. Immune-complex detachment for measurement in microvolumes on a glass microslide is performed by removal of PBS from the FT-LISA 96-well plates. Then the immune-complex degrading solution (10-50 nM NaOH + 0,5 % SDS, 12 µl) is added for a minimum of 5 minutes to detach FT-immune-complexes. Our proposed method of analysis with the use of immune-complex degrading solution and transferring samples to microvolume in amount from 0.5 to 5 µl reading format (plates/glass slides) can be used not only for single analyte detection (singleplex), but also quantification of two or more analytes (multiplex) in the same well, using different wavelength FTs. In order to evaluate the suitability of streptavidin-coated FTs for biomarker multiplex analysis, their optical properties were investigated and detection limits were determined. The single analyte detection is performed with one analyte which is immobilised on the bottom of the well, and with one FT with biotinylated antibody in the well.

The multiple analyte detection is performed with two or more analytes which are immobilised on the bottom of the well, and with two or more FT-antibody conjugates in the same well. The method was performed by using two different analytes - cartilage oligomeric protein (COMP) and human growth hormone (hGH).

The new method has shown unexpectedly improved properties for the detection of at least one analyte in the sample which is taken from the patient. Present invention enables the enhancement of the reaction sensitivity, (namely, reduces the lowest detection limit) up to forty times.

The present invention is used for the diagnostic and/or prognosis of the diseases due to the ability to detect a combination of biomarkers in the same sample with higher sensitivity. Immune-complex, comprising at least one analyte, biotinylated antibody and streptavidin-coated fluorescent tag is *in vitro* used for diagnosis and/or prognosis of disease, wherein disease is inflammatory or degenerative joint disease.

In another aspect, some of the biomarkers are not specific enough for a particular disease. But in combination with another, more specific biomarker, it can potentially lead to a better understanding of disease progression mechanisms.

### BRIEF DESCRIPTION OF THE FIGURES

The accompanying drawings, which are incorporated in and constitute a part of this specification, illustrate one embodiment of the invention and, together with the description, serve to explain the principles of the invention.
Figure 1 depicts a scheme of a conventional QLISA detection method.
Figure 2 depicts a single or multiplex detection of analytes in microvolume format.
Figure 3 depicts microvolume format for spectrophotometry. 3A- microvolume reiterative glass slide. 3B - principal scheme of microdrop formation and the need to dilute the immune-complex degrading solution.
Figure 4 depicts fluorescence intensity comparison of QD565 in different solutions based on concentration.
Figure 5 depicts fluorescence intensity comparison of 10 nM QD565 in different solutions based on time.
Figure 6 demonstrates comparison of median fluorescence intensities (MFIs) between detection of hGH in standard 96-well plate QLISA and microslide format using different wavelength QDs.
Figure 7 depicts comparison of hGH detection in a 96-well plate by QLISA on magnetic beads vs. detection of the detached QDs using a microslide.
Figure 8 depicts detection of different COMP concentrations in 96 well plate vs. microplate format.
Figure 9 depicts the detection of different hGH concentrations with anti-hGH-biotinylated Ab and streptavidin-coated QD625.
Figure 10 depicts the comparison of COMP concentrations in OA cartilage explant secretome under different conditions (mechanical load, IL-1β stimulation).
Figure 11 depicts the comparison of clusterin concentrations in OA cartilage explant secretome under different conditions (mechanical load, IL-1β stimulation).
Figure 12 depicts photoluminescent in the UV-NIR spectra CdSe/ZnS QDs. A - different size QD photoluminescence spectra; B - normalized photoluminescence spectra with excitation using 400 nm wavelength.
Figure 13 demonstrates the principal scheme of singleplex and duplex QLISA.
Figure 14 the detection of different COMP concentrations (50 nM, 25 nM, 5 nM ) in 96 well plate vs. microplate format.
Figure 15 depicts detection of different hGH concentrations in 96 well plate vs. microplate format.
Figure 16 depicts fluorescence intensity comparison of QD565 in different solutions, measured by fluorescence spectroscopy.
Figure 17 depicts dilution of immune-complex degrading solution with PBS 1:1 for the measurement of COMP on microslide.

### DESCRIPTION OF THE PREFERRED EMBODIMENT

### Abbreviations

Ab, antibody; COMP, cartilage oligomeric protein; ELISA, enzyme-linked immunosorbent assay; ECM, extracellular matrix; FRET, fluorescence resonance energy transfer; FT - fluorescent tag, definition includes quantum dots, fluorescent dyes, fluorescent microspheres; FT-LISA - FT based immunoassay; hGH, human growth hormone; HRP, horseradish peroxidase; IL-1β, interleukin 1β; MB, magnetic beads; MFI, median fluorescence intensity; OA, osteoarthritis; PBS, phosphate-buffered saline; RA, rheumatoid arthritis; QD, quantum dot; QLISA, quantum dot-linked immunosorbent assay; RT, room temperature; SDS, sodium dodecylsulfate; SPR, surface plasmon resonance; v/v, volume (of solute) per volume (of solvent).

### Definitions

As used throughout this specification, the term "analyte A or B" means two analytes that were detected in experiments included in this description.

The term " biotin-labelled anti-analyte A and B antibodies " as used herein is intended to be an antibody used for analyte A and B detection, and biotin labelling allows the antibody to bind streptavidin-coated multicolor FTs as photoluminescent QDs..

The term "immune-complex - nanoparticle structure" means analyte + biotin labelled anti-analyte detection antibodies + streptavidin-coated FTs.

"Streptavidin-coated FTs" as used throughout this specification is intended to describe modified QDs, which bind to biotin-labelled analyte A and B detection antibodies.

The term "immune-complex degrading solution" means a solution, which is used to break the connection between the analyte and biotinylated antibody. The result is transfer of biotinylated antibody - streptavidin-coated FTs complexes (detached from the bottom of the well) into the solution.

Reference will now be made in detail to the presently preferred embodiments of the invention, which together with the following examples, serve to explain the principles of the invention.

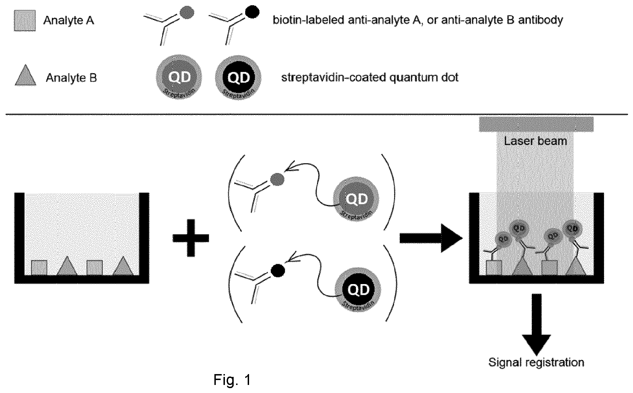

In one embodiment, the present invention relates to a method of enhancing the nanoparticle-based immunoassay (FT-LISA or QLISA) detection sensitivity through detachment of FTs or QDs from the bottom of the 96-well plate, using a specific immune-complex degrading solution and further dilution in PBS and transfer to a microplate for more sensitive measurement in microvolume quantities. In this invention, multicolor FTs as photoluminescent QDs conjugates are applied to ensure simultaneous detection of analytes A and/or B, therefore below is the QD immunoassay example. Usually in QLISAs, the analyte (namely, capture antibodies and then analytes) on the plastic surface can be quantified using biotinylated anti-analyte A detection antibodies, which are conjugated to streptavidin coated-QDs through biotin-streptavidin interaction and measured spectroscopically from the bottom of the well (Tang M et al. 2018). The QD signal intensity represents the quantity of an analyte on the bottom of the well. Analytes (namely, capture antibodies and then analytes) are attached on the bottom of the plate, incubated with detection antibodies conjugated with QDs, followed by the direct registration of their fluorescence signal from bottom of the plate (Figure 1).

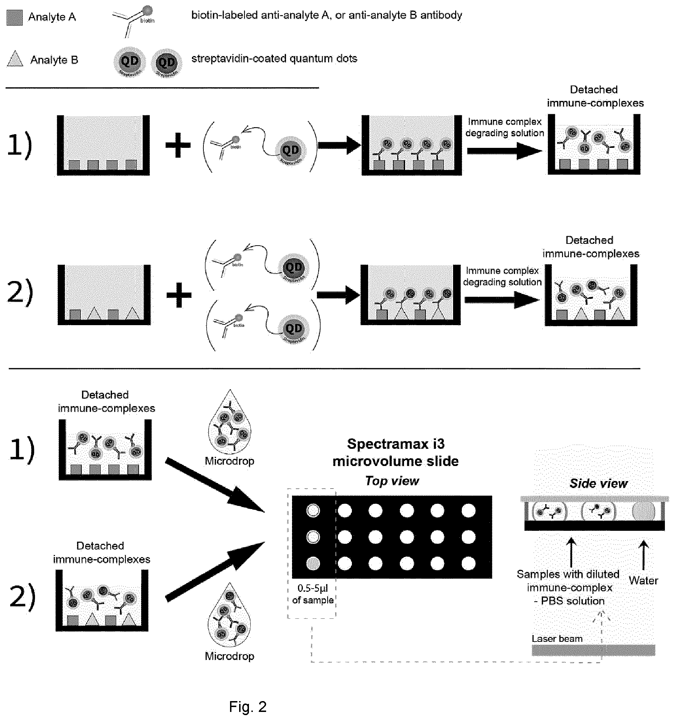

Formation of analyte A (1) or A/B (2) with biotin-labelled anti-analyte A (1) or A/B (2) antibodies and streptavidin-coated QDs are shown in Figure 2. immune-complex degrading solution dissociates antibody complexes through analyte-biotinylated antibody connection. After dissociation, microdrops are transferred to microvolume reiterative glass slide-based spectrophotometric scan.

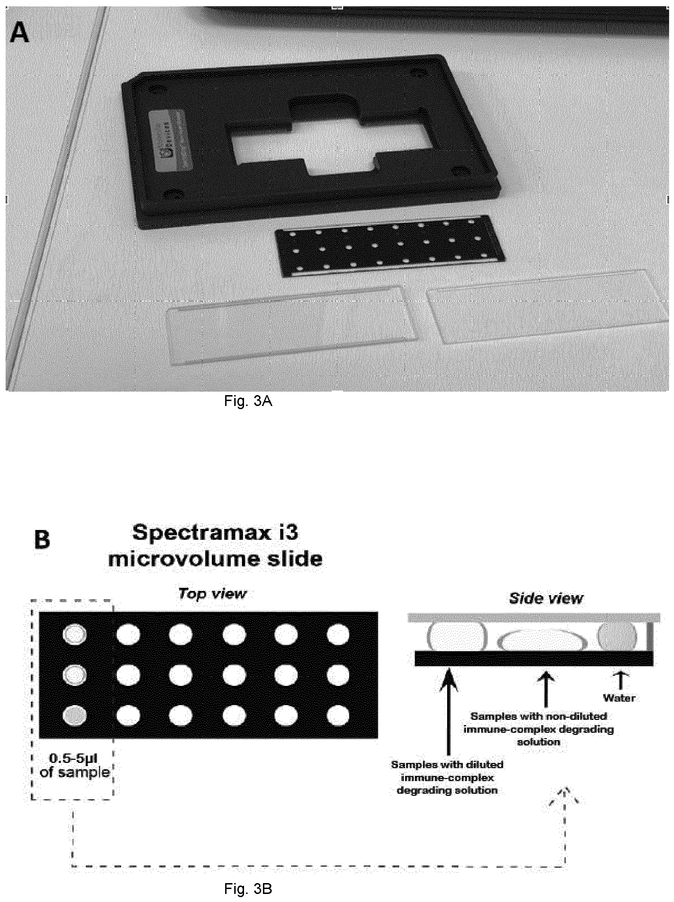

The current invention includes methods for the enhancement of detection specificity of analyte by QLISA through application of a microvolume for QD fluorescence signal registration using fluorescence spectroscopy. The present invention demonstrates how QLISA signal may be amplified by application of immune-complex degrading solution for detachment/separation of immune-complexes, additional dilution ranging from 1:1 to 1:5 (1 part immune-complex solution to 5 parts PBS) in PBS and transfer for sensitive quantitative measurement of the QD fluorescence in the immune-complex - PBS solution of a microvolume size (Figures 2 and 3A and 3B). The innovative solution is the method to use microvolumes from 0.5to 5 µl on a reiterative glass slide and analyze it using fluorescent spectroscopy / spectrophotometer. Noteworthy, the suggested method requires no sophisticated cytometers with specific sets of lasers and filters, it is suitable for a standard spectrophotometer, while the microvolume format allows cost-effective measurement of analytes.

Dilution of the immune-complex degrading solution with PBS (immune-complex solution / PBS) in ratio 1:1 - 1:5 alters surface tension and leads to formation of a round-shaped drop which ensures QD fluorescence quantitative measurement in the microvolumetric format from 0.5 to 5 µl.

The system was demonstrated by using two different analytes - cartilage oligomeric protein (COMP) and human growth hormone (hGH). 17C10 anti-COMP biotinylated antibody and anti-hGH biotinylated antibody were used. Streptavidin-coated QD565, QD605, QD625 or QD655 were used as fluorescent detection nanoparticles. immune-complex degrading solutions were tested for immune-complex degrading efficacy and maintenance of fluorescence intensity of QDs in different solutions analyzed using spectrophotometer.

**Table 1. Reagents**

| **Reagent** | **Catalog number** | **Company** |
|---|---|---|
| Cartilage Oligomeric Matrix Protein Human HEK293 | RD172080100 | BioVendor R&D |
| Cartilage Oligomeric Matrix Protein Human, Mouse Monoclonal Antibody, Clone: 17C10 | RD182080100C1-01 | BioVendor R&D |
| EZ-Link Sulfo-NHS-Biotin | 21217 A39256 | Thermo Scientific |
| EZ-Link^{™} Sulfo-NHS-Biotin | 21217 | Thermo Scientific |
| Glycine (Gly) | A1067,1000 | AppliChem |
| Hydrochloric acid | 1003171000 | Merck |
| Monoclonal Human Growth Hormone Antibody | MAB1067 | R&D Systems |
| Pierce^{™} Biotin Quantitation Kit | 28005 | Thermo Scientific |
| Polyclonal Human Growth Hormone Biotinylated Antibody | BAF1067 | R&D Systems |
| Qdot^{™} 525 Streptavidin Conjugate | Q10143MP | Thermofisher Scientific |
| Qdot^{™} 565 Streptavidin Conjugate | Q10131MP | Thermofisher Scientific |
| Qdot^{™} 625 Streptavidin Conjugate | A10196 | Thermofisher Scientific |
| Qdot^{™} 655 Streptavidin Conjugate | Q10123MP | Thermofisher Scientific |
| Human Growth Hormone Antibody | MAB1067-100 | R&D Systems |
| Recombinant Human Growth Hormone (GH) Protein | 1067-GH-025/CF | R&D Systems |
| Sodium dodecylsulfate (SDS) | 436143-100G | Sigma-Aldrich |
| Sodium hydroxide | 11358504 | Thermofisher Scientific |
| Cartilage Oligomeric Matrix Protein Human ELISA | RD194080200 | BioVendor |
| Human Clusterin Quantikine ELISA Kit | DCLU00 | R&D Systems |
| Human recombinant IL-1β | A42509 | Thermofisher Scientific |
| Human recombinant TGF- β3 | RP8600 | Thermofisher Scientific |
| DMEM 1 g/L and 4.5 g/L of glucose | 21885-025, 11960-044 | Gibco, Life Technologies |
| Insulin transferin selenium (ITS) | 51500056 | Gibco, Life Technologies |
| L-proline | P5607-25G | Carl Roth |
| Ascorbic acid phosphate | 49752-10G | Sigma Aldrich |
| Penicilin/Streptomycin | 15140122 | Gibco, Life Technologies |
| Dexamethasone | D4902-25MG | Sigma Aldrich |
| Gentamicine | G1272-100ML | Sigma Aldrich |
| GlutaMaX | 35050061 | Gibco, Life Technologies |
| Dynabeads^{™} M-280 Streptavidin | 11205D | Thermofisher Scientific |

**Table 2. Equipment**

| **Device** | **Company** |
|---|---|
| Double-channel surface plasmon resonance "Autolab Esprit" | Metrohm Autolab BV, The Netherlands |
| FLS920 Fluorescence Spectrometer | Edinburgh Instruments, United Kingdom |
| Spectrophotometer SpectraMax^{®} i3 | Molecular Devices^{®}, USA |
| Flexcell FX-5000 | Flexcell International, Hillsborough, NC |

### Methods

Antibody biotinylation method. Thermo Scientific^{™} EZ-Link^{™} Sulfo-NHS-Biotin (sulfosuccinimidyl biotin) binds to antibody primary amine groups. As a result, biotinylated antibodies are obtained. Dialysis step eliminates the residual biotinylation reagent. Antibody biotinylation efficiency is measured with a specific kit (Pierce Biotin Quantitation Kit). After these steps biotinylated antibodies can bind to streptavidin-coated QDs.

ELISA/QLISA. Firstly, the ELISA method was used to evaluate analyte-antibody binding efficiency. For the detection of immune-complex, instead of conventional HRP (horseradish peroxidase), streptavidin-coated QDs were used as fluorescent signal carriers (QLISA).

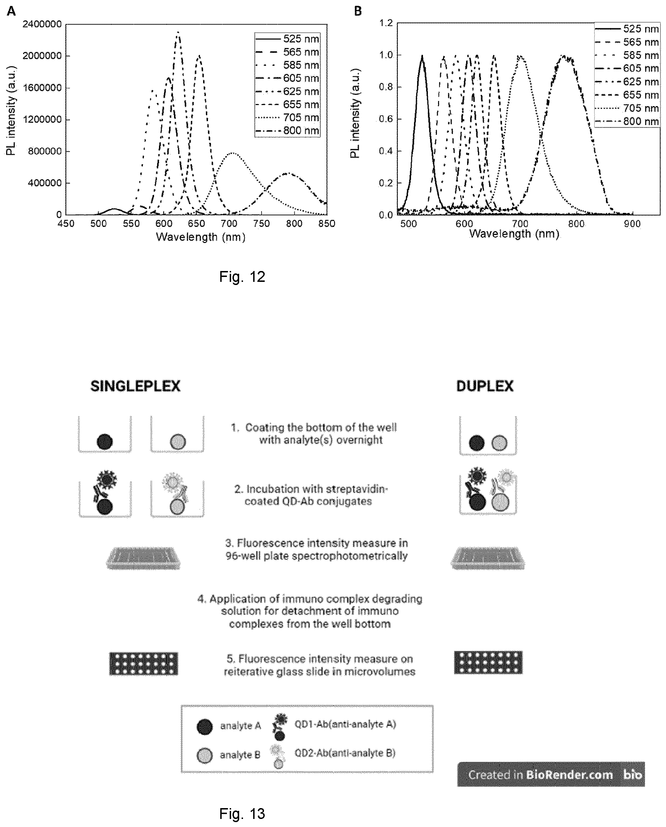

### Procedure for singleplex QLISA (Figure 2A, Figure 13):

Different analyte A concentrations are applied to 96-well plates in duplicates for overnight adsorption to the bottom of the well (50 pl/well).

Washing with PBS (3X).

Blocking with 4% BSA/PBS for 2 hours.

Washing with PBS (3X).

Applying 10 nM or 20 nM of biotinylated detection antibodies (anti-analyte A), 50 pl/well in concentrations ranging from 50 nM to 3.125 nM for 1 hour in RT (room temperature), or incubation of biotinylated detection antibodies with streptavidin-coated QDs in a separate "low protein binding" tubes for at least 1 hour.

Washing with PBS (3X).

Addition of 10 nM streptavidin-coated QDs or the mixture of diluted Ab-QD conjugates generated in "low protein binding" tubes in step 5, 50 pl/well for 1 hour.

Washing with PBS (3X).

Addition of 80 pl/well of PBS and fluorescence measurement from the bottom of the plate. Measurement parameters: excitation - 400 nm; emission - depending on the QD wavelength; spacing - 0.40; density - 9 (resulting in a total of 69 scans / well).

### Procedure for duplex QLISA (Figure 2B, Figure 13):

A solution containing two different analyte (analyte A and B) concentrations (ranging from 50 nM to 3.125 nM) are applied to 96-well plates in duplicates for overnight adsorption to the bottom of the well (50 µl/well). Meaning that for example in one well there is 50 nM analyte A and 50 nM analyte B.

Washing with PBS (3X).

Blocking with 4% BSA/PBS for 2 hours.

Washing with PBS (3X).

Applying 10 nM or 20 nM of biotinylated detection antibody mix (anti-A and anti-B), 50 pl/well for 1 hour in RT (room temperature), or incubation of biotinylated detection antibodies with different wavelength streptavidin-coated QDs in a "low protein binding" tubes for 1 hour. (one tube with biotinylated anti-analyte A and QD No.1, the second - with biotinylated anti-analyte B and QD No.2)

Washing with PBS (3X).

Addition of 10 nM streptavidin-coated QDs or the mixture of diluted Ab-QD conjugates generated in "low protein binding" tubes in step 5, 50 µl for 1 hour. (before adding, solutions from both tubes are mixed to form a one mixture of two Ab-QD conjugates in one solution) Washing with PBS (3X).

Addition of 80 µl of PBS and fluorescence measurement from the bottom of the plate. Same wells with two analyte-QD conjugates are measured at corresponding wavelengths according to the chosen QDs. Measurement parameters: excitation - 400 nm; emission - depending on the QD wavelength; spacing - 0.40; density - 9 (resulting in a total of 69 scans / well).

Both the absorption ELISA and fluorescence intensity were measured using spectrophotometer SpectraMax i3.

Human OA cartilage explant preparation. Human knee articular cartilage samples were collected from 7 patients and explants were prepared using a biopsy punch and transferred to 12 well plates. Media volumes were calculated according to explant weight. Controls were incubated in basal medium (DMEM w/o phenol red, 1% penicillin/streptomycin, 1% gentamicin and 1% GlutaMAX^{™}), and experimental samples were stimulated with interleukin 1β (IL-1β) (10 ng/ml). Explants were subjected to mechanical compression (square form dynamic compression of 30kPa 2/3, 20kPa 1/3, 1Hz, 1 hour/day) with and without additional IL-1β (10 ng/mL) stimulation. Media were changed twice weekly. Lithuanian Bioethics Committee permission from 2014-06-10 No. 158200-14-741-257.

The double channel surface plasmon resonance (SPR)-analyzer. Autolab Esprit (Metrohm Autolab BV, The Netherlands) with a small sample volume (20 - 150 ml) double channel cuvette and mixing will be used (dynamic range 4000 m°, angle resolution < 0.02 m°, change of 120 m° represents a change of 1 ng/mm2 in surface concentration of protein).

Fluorescence spectroscopy. Photoluminescence spectra of 10nM QD565 in different solutions (distilled H2O, PBS, borate-buffered saline and 50 mmol/l NaOH + 0.5 % NDS immune-complex degrading solution) were recorded with a FLS920 spectrophotometer (Edinburgh Instruments, UK). All samples were excited with 405nm, the excitation and emission slits were fixed to 5nm.

The sensitivity of the QLISA is enhanced by transfer of the well bottom-linked immune-complexes to a liquid phase using an immune-complex degrading solution (10-50 nM NaOH + 0,5 % SDS). The proposed method for degrading immune-complexes can be also applied for detection of QDs conjugated to analytes on other surfaces and/or detection systems, for instance, glass, nanoparticle or magnetic bead (MB) - bound immune-complexes.

The comparison of immune-complex degrading solution efficiency is provided in Table 3. Surface plasmon resonance results showed that the efficiency of 10 mmol/l NaOH + 0.5 % SDS as an immune-complex degrading solution was about 98.97 %, see Table 3.

**Table 3. immune-complex degrading solution efficiency.**

| **Immune-complex degrading solution** | **Degradation efficiency, %** |
|---|---|
| 10 mmol/l Gly + HCl pH 2.0 | 78.41 |
| 10 mmol/l Gly + HCl pH 1.0 | 85.72 |
| 10 mmol/l NaOH + 0.5 % SDS | 98.97 |
| 25 mM NaOH + 0.5 % SDS | 98.29 |

Plasmon resonance analysis conditions: 277.78 nM of 16F12; 19.99 nM of COMP; duration of degradation - 300 s.

Therefore, further experiments were performed using NaOH+SDS buffer.

Fluorescence intensity comparison

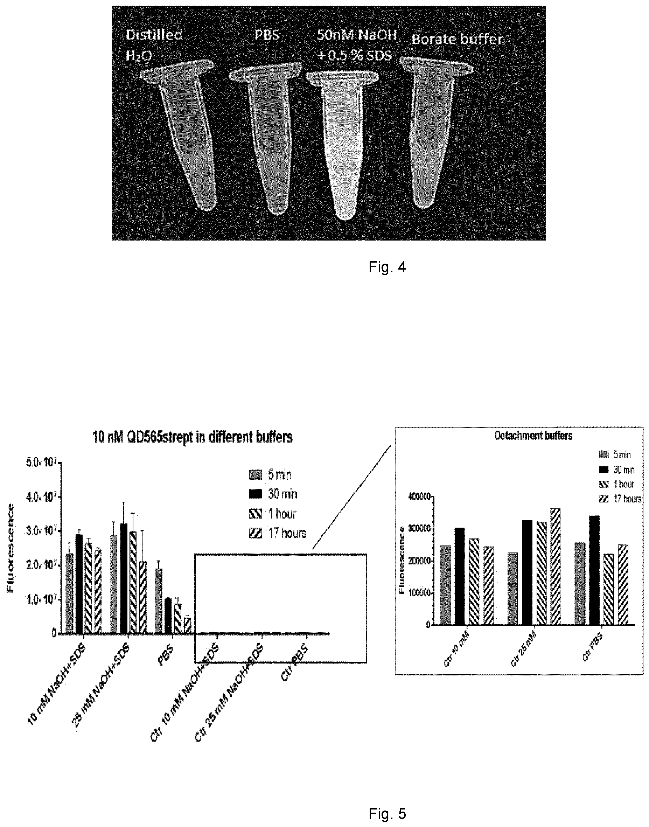

The visible differences in Figure 4 indicate that fluorescence of streptavidin-coated QD565 under UV light in 10 mM NaOH + 0.5% SDS and borate buffer is stronger than of the same amount of QD565 in PBS, distilled water.

Fluorescence of QD565 (10 nM) in NaOH+SDS buffers measured by spectrophotometer at different time points in a 96-well plate. Control (Ctr) - Ctr 10nM, Ctr 25nM, Ctr PBS - the fluorescence of the controls, 10nM and 25nM NaOH + 0.5 % SDS (immune-complex degrading solutions) and PBS respectively without the addition of QDs. Fluorescence of QD565 (10 nM) in 10 nM and 25 nM NaOH+ 0,5% SDS buffers is stronger than in PBS and more stable in time. Over a prolonged 17-hour time, the QD565 nm fluorescence signal remains sufficiently strong in these two buffers (Figure 5).

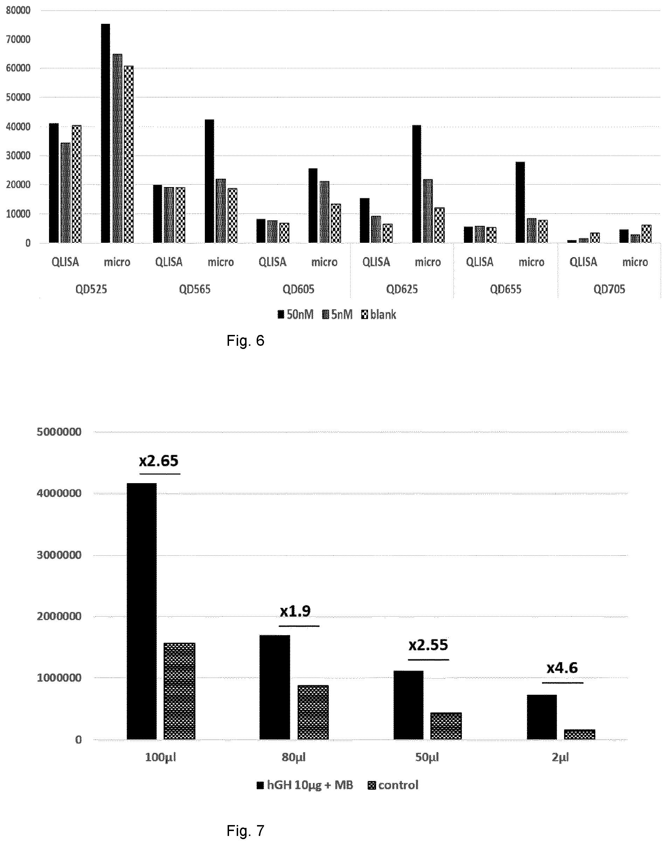

In QLISA, the immune-complex median fluorescence intensity was read with a spectrophotometer from the bottom of the well, using different wavelength absorptions (depending on the QD wavelength used for the experiment). The detachment and transfer to microvolumetric detection on a glass slide improves the signal and sensitivity. Control - every protocol step followed except the addition of analyte hGH (Figure 6).

Our proposed method for degrading immune-complexes can be also applied to QD combined with a MB detection system. Instead of measuring with flow cytometry, the samples can be analysed with a spectrophotometer in microvolumes. 100 µl 80 µl, 50 µl of MB-conjugated immune-complexes (streptavidin-coated MBs - biotinylated capture antibody - protein - biotinylated detection antibody - streptavidin-coated QDs) were analysed in 96-well plate format and after detachment of QDs-antibody complexes with 50 mmol NaOH + 0.5 % SDS on a glass microslide (0.5 - 5 µl/sample). Samples were analyzed by spectrophotometer Spectramax.

By using an immune-complex degrading solution and measurement on a glass slide we reduce autofluorescence of plastic (96-well plate) and increase the difference from blank. This step is also necessary because MB tends to sink and settle on the bottom of the well and distort the signal. Control - every protocol step followed, except the addition of analyte hGH (MBs, both antibodies and QDs) (Figure 7).

The fluorescence signal of the QD-linked immunosorbent assay (QLISA) for single and multiplex analysis is enhanced by transferring the liquid phase of degraded immune-complex - nanoparticle structure (biotin-labelled detection anti-analyte antibodies and streptavidin-coated QDs) to microvolume (2 µl) reading format (plates/glass slides) of conventional spectrophotometers.

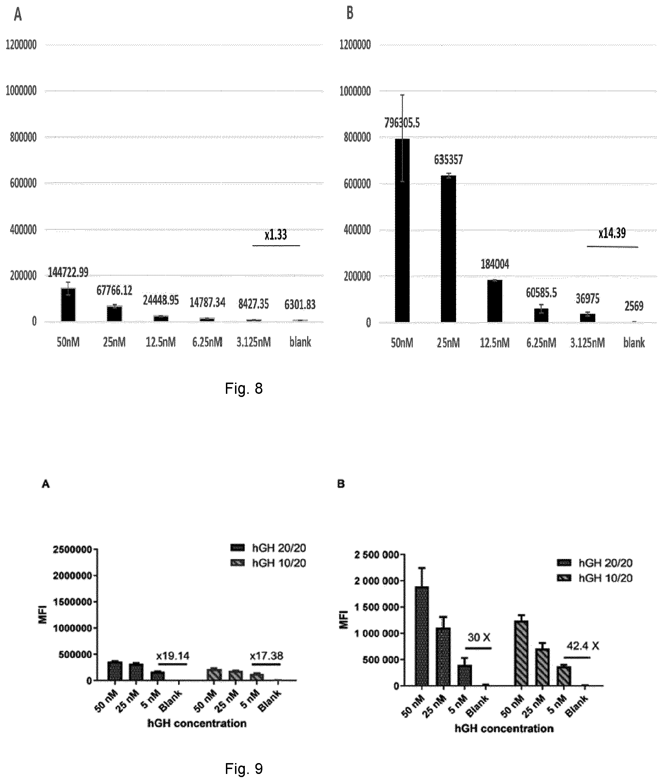

In the second embodiment QLISA provides the comparison of singleplex COMP (Figure 8) and hGH (Figure 9) detection with streptavidin-coated QD655 and QD625 respectively in 96-well plate format and microvolume format on a glass microslide after detachment with buffers. immune-complex detachment for microvolume measurement on a glass microslide is performed by removal of PBS from the QLISA 96-well plates. Then the immune-complex degrading solution (10-50 nM NaOH + 0,5 % SDS, 12 µl) is added for at least 5 minutes. After equal volume (12 µl) of PBS is added and mixed with a pipette. 2 µl of the mixture (in triplicates or more) is used for a measurement of fluorescence intensity on a glass microslide of the spectrophotometer.

17C10 anti-COMP-biotinylated Ab and streptavidin-coated QD655 were used for the detection of different COMP concentrations (50 nM to 3.125 nM) in 96-well plate vs. microplate format. A - the signal was detected from the bottom of the plate, excitation 400 nm, emission 655 nm, 80 µl/well volume. Blank - every protocol step followed without coating the bottom of the well with COMP (non-specific signal). B - the signal was detected in microvolume glass slide (2 µl/sample), excitation 400 nm, emission 655 nm. Blank - every protocol step followed without coating the bottom of the well with COMP (non-specific signal).

The average fluorescence signal of the highest COMP concentration (50 nM) was around 144723 MFI and the lowest (3.125 nM) was 8427 MFI, indicating the direct correlation between analyte concentration and fluorescence signal intensity in a 96-well plate format (Figure 8A and 8B).

The signal of the highest COMP concentration (50 nM) is 5.5 times lower in 96-well plate format plate as compared to the microvolume format in glass microslide after detachment from the bottom (144723 MFI vs. 796305 MFI respectively) (Figure 8A vs. 8B). This data validates the claim that QD-immune-complex detachment to solution, dilution in PBS and further transfer to microvolumes on microslide increases detection sensitivity, as well as decreases the background signal (blank). The difference between lowest COMP concentration tested and control blank is about 1.3 times in the 96-well plate format, while in the microslide format reaches about 14.4 times, suggesting that further lower concentrations would be detectable. Detection of different hGH concentrations (50 nM to 5 nM) with anti-hGH-biotinylated Ab (20 nM or 10 nM concentration) and streptavidin-coated QD625 (20 nM concentration) is provided in Figure 9. A - the signal was detected from the bottom of the plate, excitation 400 nm, emission 625 nm. hGH 10/20 or 20/20 represent nM concentrations of Ab/QD. Horizontal lines represent the difference between lowest hGH concentration and blank. Blank - every protocol step followed without coating the bottom of the well with COMP (non-specific signal). B - the signal was detected on a microvolume glass slide (2 µl sample size, immune-complexes degraded with 50 mM NaOH+ 0.5% SDS and diluted with PBS, 1:1 v/v). Excitation 400 nm, emission 625 nm. hGH 10/20 or 20/20 represent nM concentrations of Ab/QD. Horizontal lines represent the difference between lowest hGH concentration and blank. Blank - every protocol step followed without coating the bottom of the well with COMP (non-specific signal).

The results indicate that the use of higher anti-hGH-biotinylated Ab (20 nM) results in a stronger fluorescence intensity, compared to 10 nM. It also increases the difference between the lowest hGH concentration and blank (19.14 times vs. 17.38 times). However, use of both, 20 nM anti-hGH-biotinylated Ab/20nM streptavidin-coated QD625 and 10 nM anti-hGH-biotinylated Ab/20 nM streptavidin-coated QD625 concentrations result in a relatively strong and distinguishable between different concentration signals.

Our proposed method of analysis with the use of immune-complex degrading solution and transferring samples to microvolume (0.5 - 5 µl) reading format (plates/glass slides) can be used not only for single analyte detection (singleplex), but also quantification of two or more analytes in the same well, using different wavelength QDs.

The ability to detect a combination of biomarkers in the same sample with higher sensitivity can improve disease diagnostics. For example, the early stages of OA are asymptomatic and only changes in relevant biomarkers can indicate the disease onset. Another issue is that some of the biomarkers are not specific enough for a particular disease. For example, COMP is one of the ECM degradation biomarkers. COMP correlation in both serum and synovial fluid with OA progression has been proven with numerous studies. However, it is not specific for OA, but more applicable to various inflammatory joint diseases. But in combination with another, more specific to OA biomarker, it can potentially lead to a better understanding of disease progression mechanisms. For example, clusterin, which is thought to be involved in cytoprotective functions in osteoarticular tissues, and could be a biomarker indicating early repair responses in OA. Concentrations of both of these biomarkers, COMP and clusterin, have been separately measured using ELISA kits. The aim was to see how levels of COMP and clusterin change in different time frames, under mechanical load effect and under IL-1β stimulation (pro-inflammatory environment) in OA cartilage explant secretome.

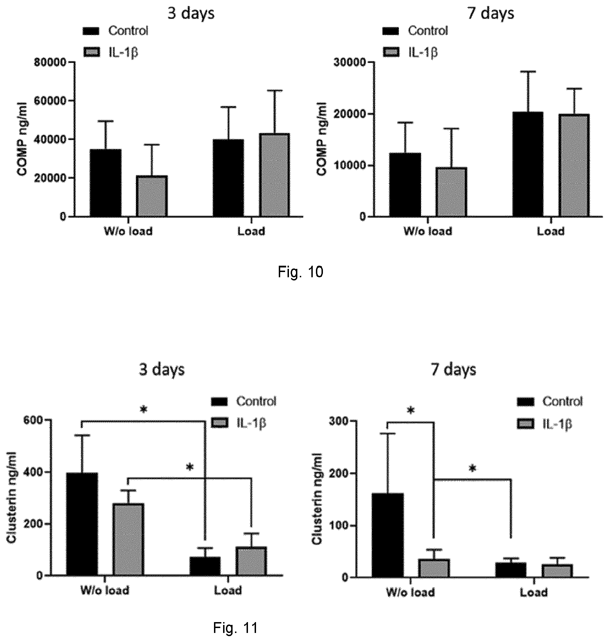

Figure 10 describes the release of COMP in OA cartilage explant secretome and the concentration changes under different conditions. Mechanical load significantly increased COMP release from IL-1β stimulated explants. After 7 days COMP levels were significantly increased after load in IL-1β stimulated samples, but not in control samples. COMP release from IL-1β-stimulated samples without load was lower.

As for clusterin, the protein released into the secretome changes under mechanical load and IL-1β stimulation are illustrated in Figure 11. After 3 days, loading significantly suppressed CLU release in IL-1β stimulated and control samples. After 7 days CLU release was decreased in IL-1β-stimulated samples without load and also in control samples. CLU expression increased following IL-1β-stimulation with and without load.

The data from COMP and clusterin experiments indicate that the combination of both markers and using them for duplex detection with QDs could result in more specific disease progression evaluation in patient samples.

In order to evaluate the suitability of streptavidin-coated QDs for biomarker multiplex analysis, their optical properties were investigated and detection limits were determined. Photoluminescent in the UV-NIR spectra CdSe/ZnS QDs of different sizes were used in the study (Figure 12). As can be seen in Figure 10, QDs with a peak at ~795 nm are not suitable for biomarker multiplexing studies because of the broad photoluminescence in the 520 - 600 nm spectral range that can enter multiple channels of fluorescence detectors. During multiplexing experiments, it is necessary to avoid photoluminescence overlap of QDs of different sizes, so it was decided to reject QDs of this size as unsuitable for the detection of multiple biomarkers.

Further investigation of streptavidin-coated QDs optical properties revealed that CdSe/ZnS QDs of different sizes exhibit different photoluminescence quantum efficiency, and the antigen detection limit will depend on the excitation wavelength and the sensitivity of the photoluminescence detector in the specific spectral range. QD detection limits were evaluated and determined: QD525 - 2 pM; QD565 - 1 pM; QD585 - 120 fM; QD605- 100 fM; QD625 - 50 fM; QD655 - 80 fM; QD705 - 200 fM; QD800 - 350 fM.

For duplex analysis in this case a combination of streptavidin-coated QD525 and QD605 was chosen. The bottom of a 96-well plate is coated with two analytes (COMP and hGH) and incubated with two QD-antibody conjugates. The principle is shown in Figure 13.

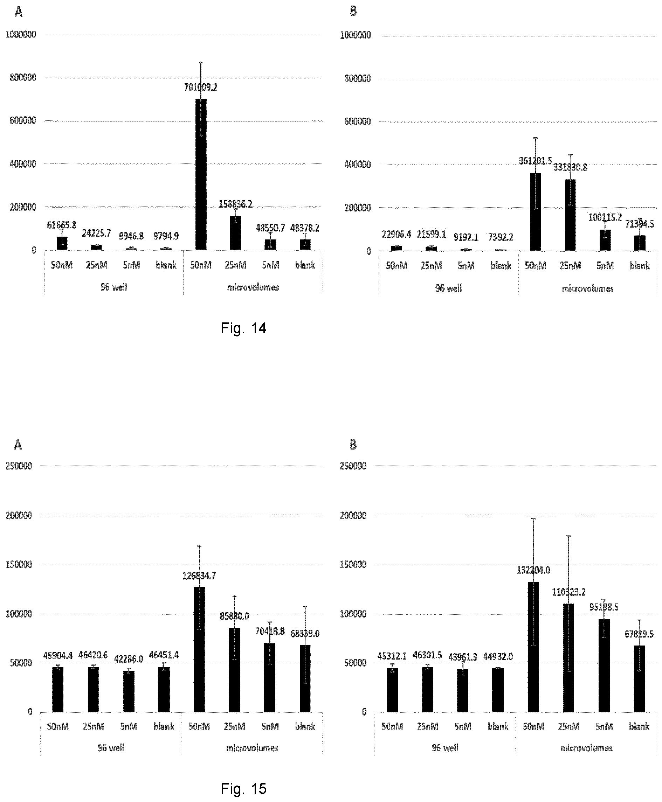

In this case, the plate was coated with 50 nM, 25nM and 5 nM of both COMP and hGH. QD525-streptavidin solution was incubated with anti-hGH antibodies, QD625-streptavidin - with anti-COMP antibodies for an hour. After 1 h, the solutions were mixed together and added 50µl to each well. Singleplex detection of each analyte was done as a comparison of fluorescence intensity (Figure 14 and 15).

Figure 14 provides the detection of different COMP concentrations (50 nM, 25 nM, 5 nM) in 96 well plate vs. microplate format. 17C10 anti-COMP-biotinylated Ab and streptavidin-coated QD605 were used. A - the comparison of singleplex COMP detection in 96-well format (signal detected from the bottom of the plate, excitation 400 nm, emission 605 nm, 80 µl volume) and in microvolumes (signal detected on a microvolume glass slide (2 pl/sample), excitation 400 nm, emission 605 nm. Blank - every protocol step followed without coating the bottom of the well with COMP (non-specific signal). B - the comparison of duplex (coating with COMP and hGH) COMP detection in 96-well format (signal detected from the bottom of the plate, excitation 400 nm, emission 605 nm, 80 µl volume) and in microvolumes (signal detected in microvolume glass slide (2µl/sample), excitation 400 nm, emission 605 nm. was detected from the bottom of the plate, excitation 400 nm, emission 655 nm, 80 µl volume. Blank - every protocol step followed without coating the bottom of the well with COMP (non-specific signal). Figure 15 provides the detection of different hGH concentrations (50 nM, 25 nM, 5 nM) in 96 well plate vs. microplate format. Anti-hGH-biotinylated Ab and streptavidin-coated QD525 were used. A - the comparison of singleplex hGH detection in 96-well format (signal detected from the bottom of the plate, excitation 400 nm, emission 525 nm, 80 µl volume) and in microvolumes (signal detected in microvolume glass slide (2µl/sample), excitation 400 nm, emission 525 nm. Blank - every protocol step followed without coating the bottom of the well with COMP (non-specific signal). B - the comparison of duplex (coating with COMP and hGH) COMP detection in 96-well format (signal detected from the bottom of the plate, excitation 400 nm, emission 605 nm, 80 µl volume) and in microvolumes (signal detected in microvolume glass slide (2µl/sample), excitation 400 nm, emission 605 nm. was detected from the bottom of the plate, excitation 400 nm, emission 655 nm, 80 µl volume. Blank - every protocol step followed without coating the bottom of the well with COMP (non-specific signal).

The results indicate that our proposed method can be used for two analyte detection in the same sample using different wavelength QDs. In 96-well format, different analyte concentrations were hardly distinguishable, but the use of the immune-complex degrading solution and transfer to microvolumetric detection increases the signal strength and can detect different analyte concentrations.

In the further embodiment, dilution of the QDs sample in the immune-complex degrading solution with PBS 1:1 v/v ensures higher measurement quality/sensitivity when measured on a glass microslide.

Although the dilution with PBS 1:1 v/v reduces fluorescence intensity of QDs, the signal becomes higher in the diluted samples when measured on microslide.

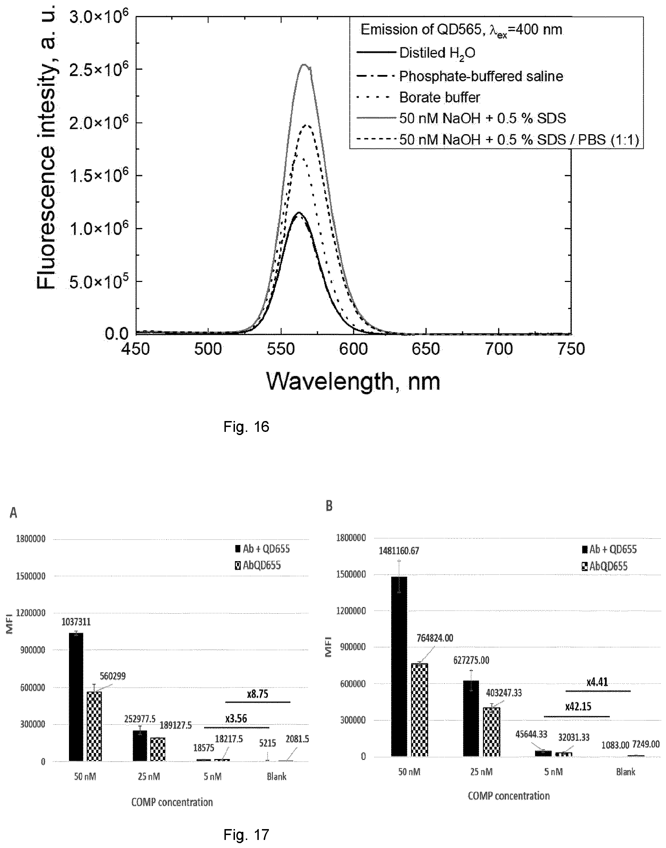

Fluorescence intensity comparison of 10nM QD565 in different solutions is measured by fluorescence spectroscopy. Fluorescence intensity comparison of 10nM QD565 showed that 50 mmol/l NaOH + 0.5 % SDS immune-complex degrading solution amplifies QD fluorescence, compared to other commonly used solutions (distilled H2O, PBS, borate-buffered saline) (Figure 16). The signal is reduced by the dilution of the initially detached samples with PBS 1:1 v/v when measured on microslide. This data indicates that 50nM NaOH + 0.5% SDS, which is the immune-complex degrading solution mentioned previously, amplifies QD fluorescence, compared to other commonly used solutions.

Even though fluorescence spectrometry has shown that fluorescence intensity is higher in non-diluted solution, the dilution with PBS is necessary to avoid foaming and to ensure the formation of round-shaped microdrop (Figure 2 and 17B). Increased fluorescence intensity in diluted samples in PBS 1:1 is related to the ability of the solution to form a round-shaped drop for measurement on a glass microslide (Figure 17A, Figure 17B). Noteworthy, stepwise analyte incubation with detection Ab followed by addition of streptavidin-coated QDs fluorescence intensity in the 96 well plate generates stronger QD signal, as compared to analyte incubation with separately prepared Ab-QD655 conjugate mixture.

Immune-complex degrading solution was diluted with PBS 1:1 for the measurement of COMP on microslide (Figure 17). COMP concentrations (50 nM to 5 nM) with 17C10 anti-COMP-biotinylated Ab and streptavidin-coated QD655. Stepwise analyte incubation with detection Ab and then addition of streptavidin-coated QDs - blue bars; analyte incubation with separately prepared Ab-QD655 conjugate mixture in the "low protein binding" tubes - orange bars; COMP detection after incubation with A - non-diluted 10 mM NaOH + 0,5% SDS buffer; B - 10 mM NaOH+ 0,5% SDS buffer diluted with PBS (1:1 v/v) on a glass microslide. MFI - median fluorescence intensity; Blank - sample without COMP protein coating but followed by all the other steps of method.

### Bibliography

Bernotiene E, Bagdonas E, Kirdaite G, Bernotas P, Kalvaityte U, Uzieliene I, et al. Emerging technologies and platforms for the immunodetection of multiple biochemical markers in osteoarthritis research and therapy. Front Med (Lausanne). 2020 Oct 21;7:572977
Bilan R, Fleury F, Nabiev I, Sukhanova A. Quantum dot surface chemistry and functionalization for cell targeting and imaging. Bioconjug Chem. 2015 Apr 15;26(4):609-624
Grabolle, M., Spieles, M., Lesnyak, V., Gaponik, N., Eychmüller, A., Resch-Genger, U., 2009. Determination of the fluorescence quantum yield of quantum dots: suitable procedures and achievable uncertainties. Anal. Chem. 81, 6285-6294. doi:10.1021/ac900308v
Jarockyte G, Karabanovas V, Rotomskis R, Mobasheri A. Multiplexed nanobiosensors: current trends in early diagnostics. Sensors (Basel). 2020 Dec 2;20(23)
Jiménez-López, J., Llorent-Martinez, E.J., 2020. Graphene quantum dots-silver nanoparticles as a novel sensitive and selective luminescence probe for the detection of glyphosate in food samples. Talanta.
Lakowicz, J.R., 2006. Principles of Fluorescence Spectroscopy, 3rd ed. Springer US, Boston, MA. doi:10.1007/978-0-387-46312-4
Liu J, Sun L, Zhan H, Fan L-J. Preparation of Fluorescence-Encoded Microspheres Based on Hydrophobic Conjugated Polymer-Dye Combination and the Immunoassay. ACS Appl Bio Mater. 2019 Jul 15;2(7):3009-3018
Mansur, H.S., Mansur, A.A.P., Soriano-Araújo, A., Lobato, Z.I.P., de Carvalho, S.M., Leite, M. de F., 2015. Water-soluble nanoconjugates of quantum dot-chitosan-antibody for in vitro detection of cancer cells based on "enzyme-free" fluoroimmunoassay. Mater. Sci. Eng. C Mater. Biol. Appl. 52, 61-71. doi:10.1016/j.msec.2015.03.022
Mobasheri A, van Spil WE, Budd E, Uzieliene I, Bernotiene E, Bay-Jensen A-C, et al. Molecular taxonomy of osteoarthritis for patient stratification, disease management and drug development: biochemical markers associated with emerging clinical phenotypes and molecular endotypes. Curr Opin Rheumatol. 2019 Jan;31(1):80-89
Song M, Mengqun Yu, Yunyun Wang, Weihua Hu, Dan Cheng, Mark T. Swihart, Yang Song, Multi-color quantum dot-based fluorescence immunoassay array for simultaneous visual detection of multiple antibiotic residues in milk, Biosensors and Bioelectronics, 2015 (72): 320-325, ISSN 0956-5663, https://doi.org/10.1016/j.bios.2015.05.018.
Suzuki, M., Udaka, H., Fukuda, T., 2017. Quantum dot-linked immunosorbent assay (QLISA) using orientation-directed antibodies. J. Pharm. Biomed. Anal. 143, 110-115. doi:10.1016/j.jpba.2017.05.014
Tobias, A.K., Jones, M., 2019. Metal-Enhanced Fluorescence from Quantum Dot-Coupled Gold Nanoparticles. J. Phys. Chem. C 123, 1389-1397. doi:10.1021/acs.jpcc.8b09108
Zhu, X., Duan, D., Publicover, N.G., 2010. Magnetic bead-based assay for C-reactive protein using quantum-dot fluorescence labeling and immunoaffinity separation. Analyst.

## Claims

1. In vitro method for fluorescence-based detection of at least one analyte by spectroscopy in a sample obtained from a subject, comprising:
(i) providing a sample comprising at least one target analyte to a plate well bottom, resulting in analyte coating;
(ii) contacting coated analyte with biotinylated detection antibody, wherein said biotinylated detection antibody allows to bind the formed immune-complex with streptavidin-coated fluorescent tags or nanoparticle;
(iii) forming an immune-complex comprising at least one analyte, biotinylated antibody and streptavidin-coated fluorescent tags on the bottom of the well;
(iv) applying the immune-complex with the degrading solution for the detachment of fluorescent tags from the bottom well plate of immune-complex, which comprises at least one analyte, biotinylated antibody - streptavidin-coated fluorescent tags complexes;
(v) diluting of the immune-complex with a buffer or diluent;
(vi) transferring the diluted immune-complex - PBS solution to a microplate in microvolume quantities for the assessment of at least one analyte using fluorescence spectroscopy.

2. The method according to claim 1, wherein the fluorescent tags are selected from the group, comprising quantum dots, fluorescent dyes, and fluorescent microspheres.

3. The method according to claim 1 or 2, wherein the analyte is first incubated with biotinylated detection antibody and then fluorescent tags conjugates on the bottom of the well.

4. The method according to any of claims 1 - 3, wherein fluorescence measurement of analyte is performed from the bottom of the plate based on (i) - (iii) steps.

5. The method according to any of claims 1 - 4, wherein the transferring of the well bottom-linked immune-complexes to a liquid phase is performed using an immune-complex degrading solution.

6. The method according to claim 5, wherein immune-complex degrading solution is NaOH + SDS solution.

7. The method according to any of claims 1-6, wherein the method comprises a step of dilution of the immune-complexes with buffer or diluent and transferring of said complexes on a reiterative glass microslide to microvolume size.

8. The method according to claim 7, wherein dilution of the immune-complex degrading solution is performed with PBS in ratio 1:1 - 1:5 v/v.

9. The method according to any of claims 1-8, wherein the dilution of the immune-complex in degrading solution with PBS enables the formation of round-shaped microdrop of the immune-complex, wherein the drop must be stretched between two glass surfaces.

10. The method according to any of claims 1-9, wherein the FT-immune-complex - PBS solution is used in a microvolume size of 0.5 - 5 µl for fluorescence quantitative measurement.

11. The method according to any of claims 1-10, wherein fluorescence measurement of analyte is performed in microvolume size on glass microslide based on (i) - (vi) steps.

12. The method according to any of claims 1-11, wherein said method is applied to both singleplex and multiplex analyte detection formats.

13. The method according to any of claims 1-12, wherein the singleplex detection is performed with one analyte, one biotinylated detection antibody, one streptavidin-coated fluorescent tag.

14. The method according to any of claims 1-12, wherein the multiplex detection is performed with two or more analytes, two or more biotinylated detection antibodies, two or more streptavidin-coated different fluorescent tags, and quantified in the same sample.

15. In vitro use of FT-immune-complex, comprising at least one analyte, biotinylated antibody and streptavidin-coated fluorescent tag, for diagnosis and/or prognosis of the disease, wherein disease is inflammatory or degenerative joint disease.
